# FASCICULE DE BREVET EUROPEEN

(11) **EP 1 075 225 B1**
(45) Date de publication et mention de la délivrance du brevet: **08.12.2004**
(21) Numéro de dépôt: 99916957.6
(22) Date de dépôt: 29.04.1999
(51) Int. Cl.: A61B 17/70

(54) **SYSTEME D'OSTEOSYNTHESE RACHIDIENNE AVEC BRIDE ET VERROU**
VORRICHTUNG ZUR WIRBELSÄULENOSTEOSYNTHESE MIT KLAMMER UND VERRIEGELUNG
BACKBONE OSTEOSYNTHESIS SYSTEM WITH COLLAR AND LOCK

(30) Priorité: 30.04.1998 FR 9805525; 22.01.1999 FR 9900703
(43) Date de publication de la demande: 14.02.2001
(73) Titulaire: Dimso Distribution Medicale du Sud Ouest, 33610 Cestas (FR)
(72) Inventeur: BACCELLI, Christian, F-33650 Saint-Médard-d'Eyrans (FR); GAUCHET, Fabien, F-60800 Duvy (FR); VIENNEY, Cécile, F-33000 Bordeaux (FR)
(74) Mandataire: Texier, Christian
(86) Numéro de dépôt international: PCT/FR1999/001021
(87) Numéro de publication internationale: WO 1999/056652

(56) Documents cités:
- EP-A- 0 682 918
- DE-A- 4 240 223
- DE-A- 19 605 640
- US-A- 5 536 268

## Description

L'invention concerne les systèmes d'ostéosynthèse rachidienne.

On connaît d'après le document WO 92/03100, en figure 6, un système d'ostéosynthèse comprenant une vis pédiculaire à deux branches aptes à recevoir entre elles une tige de liaison. Un capuchon cylindrique présente sur sa face interne un filet apte à coopérer avec un filet d'une face cylindrique externe des branches pour le vissage du capuchon sur celles-ci. Un verrou monté mobile à rotation sur le capuchon peut venir serrer la tige entre les branches lorsque le capuchon est vissé sur celles-ci.

Le document DE-196 05 640 divulgue aux figures 4 or 5 un système conforme au préambule de la revendication 1.

Un but de l'invention est de fournir un système d'un type différent de celui divulgué dans le document WO 92/03100 et notamment qui procure un assemblage du verrou et de la bride simple à réaliser et rapide à effectuer.

En vue de la réalisation de ce but, on prévoit selon l'invention un système conforme à la revendication 1.

Ainsi, on réalise le montage au moyen d'un nombre réduit de pièces, ce qui simplifie et accélère l'assemblage. De plus, la fixation des deux pièces est stable et sûre.

Avantageusement, la bride et le verrou présentent des filets d'introduction pour introduire dans la gorge l'autre parmi la bride et le verrou.

Avantageusement, la bride et/ou le verrou est déformable élastiquement pour introduire dans la gorge l'autre parmi la bride et le verrou.

Ainsi, il est très facile d'assembler le verrou et la bride pour obtenir le montage précité, sans toutefois nuire à la qualité et à la stabilité de la liaison obtenue.

Avantageusement, le verrou comprend au moins une fente au niveau de la gorge.

Avantageusement, la bride s'étend en regard du filet de serrage du verrou.

Ainsi, la bride maintient les branches en position exactement en regard du filet. Tout écartement ou rapprochement des branches est donc exclu, quels que soient les efforts tendant localement à déplacer les branches.

Avantageusement, le filet de serrage du verrou est l'un des filets d'introduction.

Ainsi, on évite d'avoir à réaliser de nombreux filets. Par conséquent, on peut augmenter les dimensions du filet et donc faciliter sa fabrication sans augmenter comparativement les dimensions de la pièce.

Avantageusement, le système est configuré de sorte que le filet de serrage s'étende entre la gorge et la tige.

Avantageusement, le système est configuré de sorte que la gorge s'étende entre la tige et le filet de serrage.

Avantageusement, la bride comprend un bras apte à s'étendre entre les branches parallèlement à la tige et à participer au montage par gorge du verrou sur la bride.

Avantageusement, le bras comprend deux parties aptes à s'étendre de part et d'autre du verrou.

Avantageusement, les deux parties sont espacées l'une de l'autre.

Avantageusement, au moins une des deux parties comprend une fente.

Avantageusement, les deux parties sont déformables élastiquement.

Avantageusement, les deux parties sont reliées l'une à l'autre.

On prévoit également selon l'invention une combinaison d'une bride et d'un verrou pour un système d'ostéosynthèse selon l'invention, la bride comprenant un collier, le verrou étant apte à être monté mobile à rotation sur la bride, dans lequel l'un parmi la bride et le verrou présente une gorge apte à recevoir l'autre parmi la bride et le verrou pour le montage du verrou sur la bride, la bride et le verrou présentant des moyens de montage pour introduire dans la gorge l'autre parmi la bride et le verrou.

D'autres caractéristiques et avantages de l'invention apparaîtront encore dans la description suivante de six modes préférés de réalisation donnés à titre d'exemples non limitatifs. Aux dessins annexés :
- la figure 1 est une vue en perspective éclatée du verrou et de la bride selon un premier mode de réalisation de l'invention ;
- la figure 2 est une vue de dessus de la bride et du verrou de la figure 1 assemblés ;
- la figure 3 est une vue en coupe axiale du verrou et de la bride assemblés selon le plan III-III de la figure 2 ;
- la figure 4 est une vue analogue à la figure 3 montrant le verrou et la bride sur une vis pédiculaire ;
- la figure 5 est une vue analogue à la figure 1 montrant un deuxième mode de réalisation ;
- la figure 6 est une vue de dessous du verrou et de la bride de la figure 5 assemblés ;
- la figure 7 est une vue en coupe axiale de la bride et du verrou selon le plan VII-VII de la figure 6 ;
- la figure 8 est une vue analogue à la figure 1 montrant un troisième mode de réalisation ;
- la figure 9 est une vue éclatée en perspective de dessus d'un quatrième mode de réalisation préféré ;
- la figure 10 est une vue éclatée en perspective de dessous du quatrième mode de réalisation ;
- la figure 11 est une vue en coupe axiale du quatrième mode de réalisation monté sur un organe d'ancrage ;
- les figure 12 et 13 sont une vue de dessous et une vue en coupe axiale selon XIII-XIII d'un cinquième mode de réalisation ; et
- la figure 14 est une vue éclatée en perspective d'un sixième mode de réalisation.

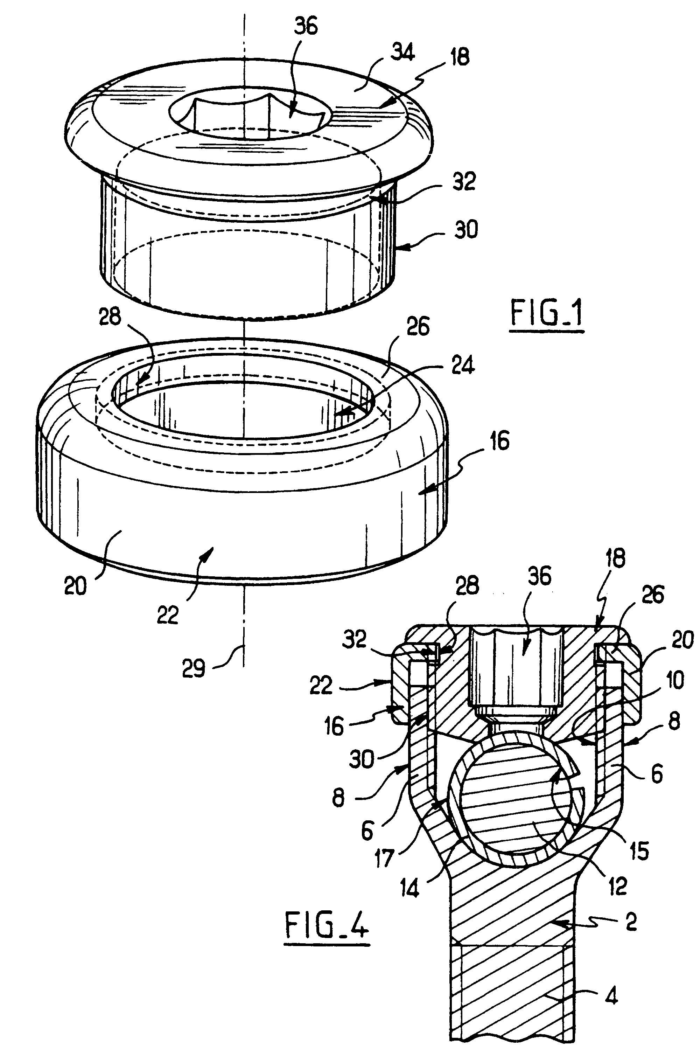

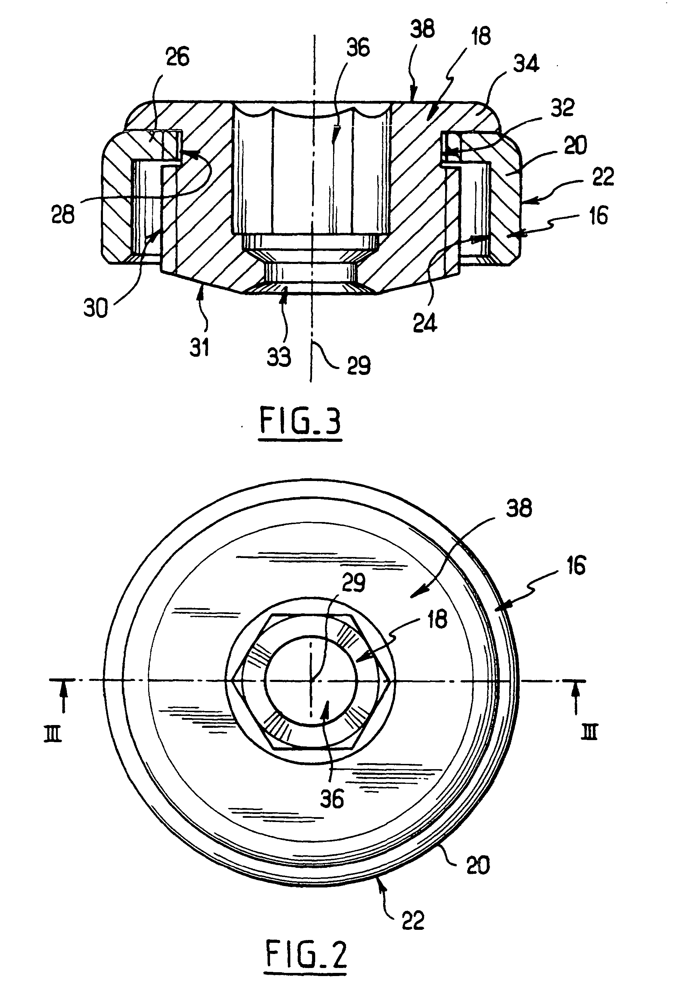

On va décrire un premier mode de réalisation du système d'ostéosynthèse rachidienne selon l'invention en référence aux figures 1 à 4.

Le système comprend des organes d'ancrage vertébraux ici sous la forme de vis pédiculaires 2 comprenant chacune un corps fileté 4 et une tête à deux branches 6 s'étendant à distance et en regard l'une de l'autre. Les deux branches 6 constituent deux secteurs d'un même cylindre coaxial au corps 4 de la vis. Les faces externes cylindriques 8 des branches sont coaxiales et lisses. Les faces internes cylindriques 10 des branches sont coaxiales et filetées. Le système comporte une tige allongée rigide lisse 12 à section transversale circulaire, et pour chaque vis 2, une bague ou olive 14 à face interne cylindrique 15 sensiblement de même rayon que celui de la tige 12 et à face externe sphérique convexe 17 de même rayon qu'un siège sphérique concave de la tête de vis entre les branches 6. La bague 14 est fendue sur un côté parallèlement à son axe et peut être reçue sur la tige 12 par ajustement glissant. Cet assemblage permet de régler l'orientation de la tige 12 par rapport à la vis 2 dans deux plans perpendiculaires entre eux, autour d'une position moyenne de la tige. On trouvera plus de détails sur ces éléments dans les documents FR-2 642 643 et FR-2 659 546 incorporés ici par référence.

Pour chaque vis 2, le système comprend une bride 16 et un verrou 18. La bride 16 comporte une paroi latérale 20 constituant un collier ou bague, à faces externe 22 et interne 24 cylindriques, ici lisses. La face interne 24 a un rayon environ égal à celui de la face externe 8 des branches 6, pour réaliser un ajustement glissant sur celles-ci. La bride 16 est ici un capuchon et comporte à un bord supérieur du collier 20 une paroi supérieure plane 26 perpendiculaire à l'axe 29 du collier et présentant en son centre un orifice 28 muni d'un filet que nous appellerons ici filet d'introduction.

Le verrou 18 présente une face externe cylindrique 30 d'axe 29 munie, sur toute sa longueur et notamment à partir d'une face inférieure 31 du verrou, d'un filet d'introduction apte à former une liaison vis-écrou avec celui 28 de la bride. Le verrou 18 présente une gorge annulaire 32 à profil en "U" dans laquelle débouche la face cylindrique filetée 30. Cette gorge a, parallèlement à l'axe 29, une largeur légèrement supérieure à l'épaisseur de la paroi supérieure 28 de la bride, de sorte que cette paroi 28 peut être reçue dans la gorge 32. Du côté de la gorge 32 opposé à la face cylindrique 30, le verrou présente une paroi supérieure plane circulaire 34 perpendiculaire à l'axe 29 et de rayon supérieur à celui de l'orifice 28 de la bride. Une empreinte hexagonale six-pans 36 est ménagée dans le verrou à partir de sa face supérieure 38. Le verrou comme la bride sont généralement symétriques de révolution autour de leur axe 29.

Le verrou 18 et la bride 16 étant initialement séparés comme sur la figure 1, pour les assembler on engage la face inférieure 31 du verrou dans l'orifice 28 de la bride pour mettre en prise mutuelle les deux filets d'introduction 28 et 30. On effectue le vissage jusqu'à introduire la paroi 26 de la bride dans la gorge 32 du verrou. La prise mutuelle des filets d'introduction 28, 30 cesse alors et la bride est rendue mobile à rotation par rapport au verrou autour de leur axe commun 29 en étant bloquée à coulissement par rapport au verrou. Dans cette position, la paroi latérale 20 de la bride s'étend sur toute sa longueur en regard et à distance du filet d'introduction 30 du verrou. Les filets d'introduction 28, 30 sont ici agencés de sorte que le filet d'introduction 30 du verrou peut venir en prise avec le filet de serrage 10 des branches 6 et fait donc office de filet de serrage. Par conséquent, le filet d'introduction 28 de la bride a les mêmes grandeurs caractéristiques que celui des branches 6.

Lors d'une intervention, on installe chaque vis pédiculaire 2 sur une vertèbre, puis on loge la tige 12 avec la bague 14 entre les branches 6. Le verrou 18 et la bride 16 ayant été préalablement assemblés comme précité, on amène la bride 16 à l'extrémité supérieure des branches 6 et on visse le verrou 18 entre celles-ci, le filet d'introduction 30 du verrou coopérant avec le filet de serrage 10 des branches 6. Au fur et à mesure de la descente du verrou 18, la bride 16 suit ce dernier en coulissant sur la face externe 8 des branches 6. L'extrémité inférieure 31 du verrou, dont la portion la plus basse est une arête circulaire délimitant une face sphérique concave 33, vient en contact avec la face externe sphérique convexe 17 de la bague 14 avant que des bords supérieurs d'extrémité des branches 6 ne viennent en butée contre la face inférieure de la paroi supérieure 26 de la bride. Grâce à la bride qui interdit l'écartement mutuel des branches 6, on serre alors le verrou 18 sur la bague 14 pour bloquer la tige 12 dans la bague dans l'orientation angulaire choisie au fond de la tête de la vis pédiculaire.

Dans le deuxième mode de réalisation illustré aux figures 5 à 7, la paroi supérieure 26 de la bride est remplacée par un bras 26a de forme générale plane, rectangulaire, s'étendant perpendiculairement à l'axe de la bride diamétralement d'un bord à l'autre de sa paroi latérale 20. Le bras 26a est contigu à un bord inférieur de la paroi latérale 20. Il présente l'orifice fileté 28 en son centre. Le bras 26a délimite ainsi deux ouvertures 42 ayant un bord interne rectiligne formé par le bras 26a et un bord externe circulaire formé par la paroi cylindrique 20 de la bride, ici assimilable à une bague. Ces ouvertures 42 sont aptes à recevoir les branches 6 respectives, entre lesquelles le bras peut donc lui-même être inséré parallèlement à la tige 12.

Sur le verrou 18, la face cylindrique externe filetée 10a s'étend maintenant à partir de la face supérieure 38 du verrou jusqu'à la gorge 32 située sous celle-ci. La paroi supérieure 34 est remplacée par une paroi inférieure 40 contiguë à la gorge dont elle forme un flan. Le rayon de cette paroi inférieure 40 est supérieur à celui du fond de la gorge 32 et inférieur à celui de la face cylindrique externe 10a. La paroi inférieure 40 porte sur son pourtour un filet d'introduction 30 apte à coopérer avec celui 28 de la paroi inférieure 26a de la bride. Ces filets d'introduction 28, 30 ont ici un rayon différent de ceux des filets de serrage 10 des branches 6 et 10a du verrou, de sorte qu'ils ne peuvent pas coopérer avec ceux-ci.

Pour assembler la bride 16 et le verrou 18, on introduit le verrou par sa paroi inférieure 40 en regard de la paroi latérale cylindrique 20 de la bride jusque dans l'orifice 28 du bras 26a. On visse alors le verrou dans cet orifice jusqu'à loger le bras dans la gorge 32 et obtenir un assemblage analogue à celui décrit dans le premier mode.

On met en place cette combinaison sur la vis pédiculaire 2 en introduisant les bords supérieurs d'extrémité des branches 6 dans les ouvertures 42. Les filets de serrage 10 des branches viennent alors en prise avec celui 10a du verrou. On manoeuvre donc le verrou pour le faire descendre en direction de la tige 12, avec la bride 16 dont la paroi latérale cylindrique 20 suit le verrou à l'extérieur des branches 6 et le bras 26a suit le verrou entre les branches. A un certain stade, les branches 6 peuvent émerger en saillie des parties supérieures de la bride et du verrou, entre ceux-ci. La gorge 32 est cette fois entre le filet de serrage 10a du verrou et la tige 12. Le serrage de l'ensemble s'achève comme dans le premier mode.

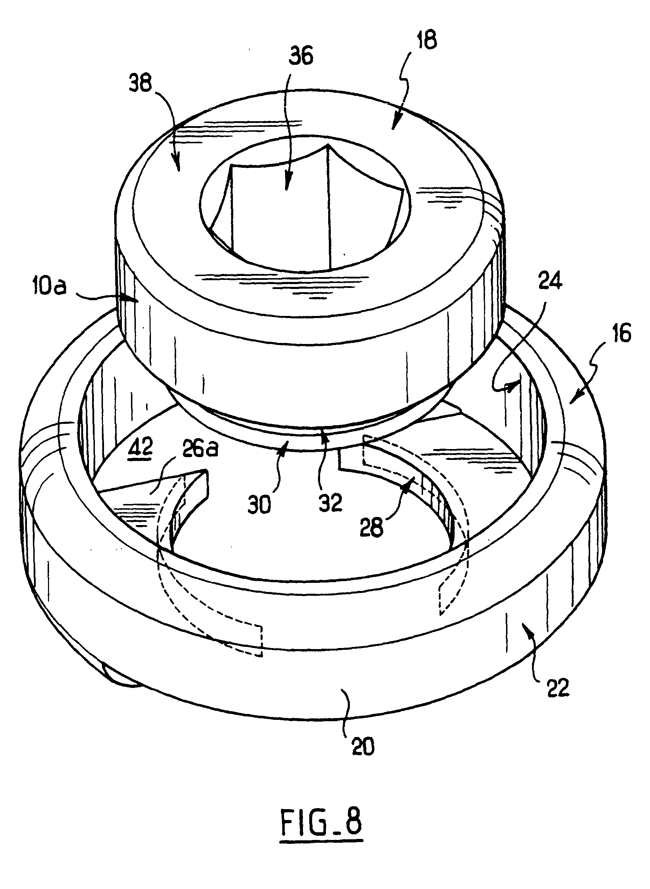

Le troisième mode de réalisation illustré à la figure 8 constitue une variante du deuxième mode. Ici, le diamètre de l'orifice 28 du bras 26a est supérieur à la largeur du bras de l'une à l'autre des ouvertures 42. Les deux portions du bras 26a situées de part et d'autre de l'axe de la bride ne sont donc plus reliées l'une à l'autre comme sur la figure 5, mais espacées l'une de l'autre. L'orifice 28 communique cette fois avec les deux ouvertures 42. Le fonctionnement du système pour l'assemblage de la bride avec le verrou et leur montage sur la vis est inchangé.

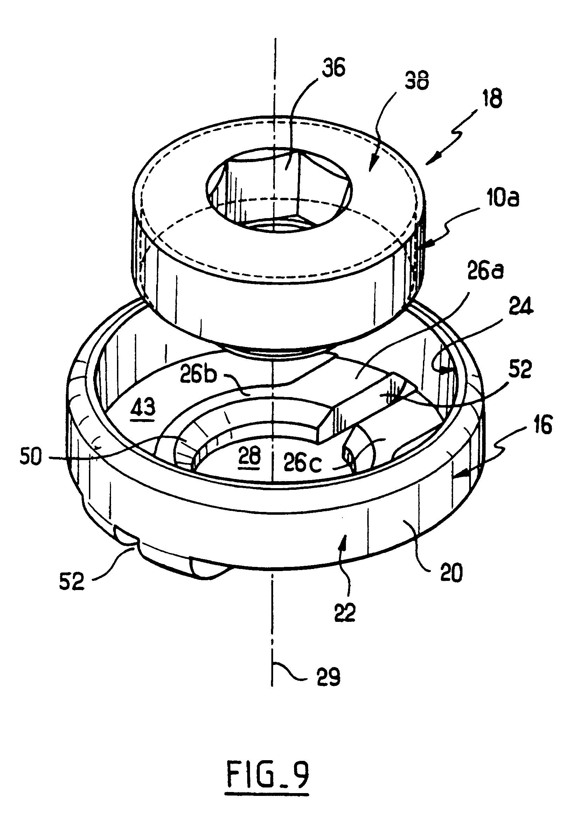

Dans un quatrième mode de réalisation du système d'ostéosynthèse rachidienne selon l'invention en référence aux figures 9 à 11, la bride 16 comporte un bras 26a semblable à celui du deuxième mode de réalisation précité, en deux parties 26b et 26c. Il présente un orifice lisse 28 comportant un chanfrein d'introduction 50. Une fente 52 sépare les deux parties 26b et 26c du bras 26a. Cette fente 52 est parallèle à l'axe médian du bras 26a et est placée de manière à ce que les parties 26b et 26c soient symétriques l'une de l'autre en miroir. Le bras 26a délimite ainsi deux ouvertures 43 ayant un bord externe circulaire formé par la paroi cylindrique 20 de la bride 16 qui est ici assimilable à une bague, et un bord intérieur présentant deux faces planes 51 séparées par une face circulaire 53 coaxiale à la paroi cylindrique 20. Ces ouvertures 43 sont aptes à recevoir les branches 6 respectives, entre lesquelles le bras peut donc lui-même être inséré parallèlement à la tige 12.

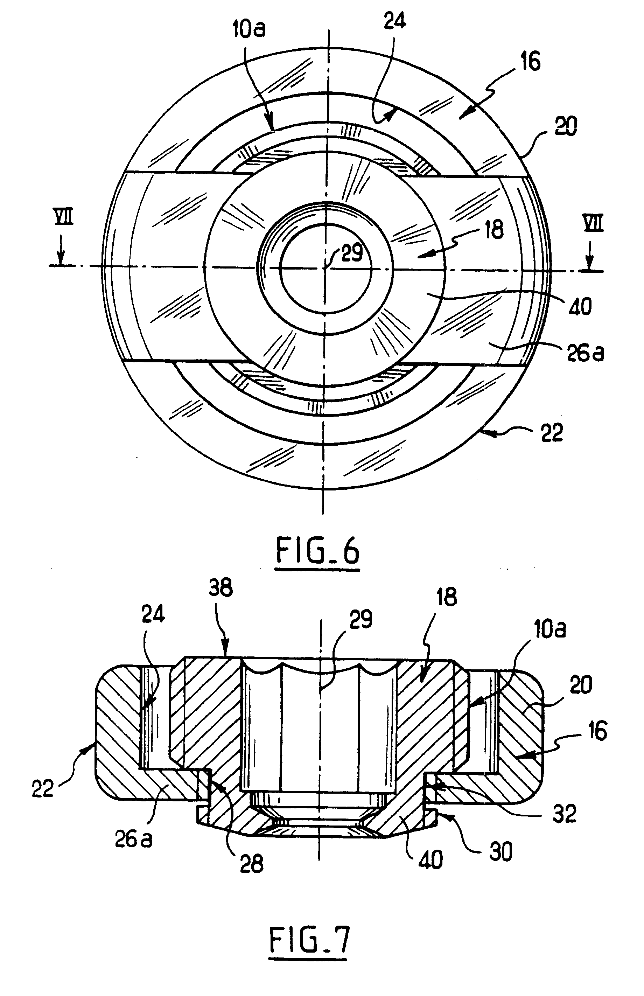

De même que dans le mode de réalisation de la figure 7, le verrou 18 présente une lèvre 60, dite lèvre d'introduction, non filetée s'étendant en saillie du fond de la gorge et surmontée de la gorge annulaire 32 à profil en « U ». La forme de la lèvre 60 est complémentaire du chanfrein d'introduction 50 de l'orifice 28 de la bride 16. La gorge 32 a, parallèlement à l'axe 29, une largeur légèrement supérieure à l'épaisseur du bras 26a de la bride, de sorte que ce bras peut être reçu dans la gorge 32. Du côté de la gorge 32 opposé à la lèvre d'introduction 60, le verrou présente une paroi supérieure 54 plane, circulaire perpendiculaire à l'axe 29 et de rayon supérieur à l'orifice 28 de la bride 16.

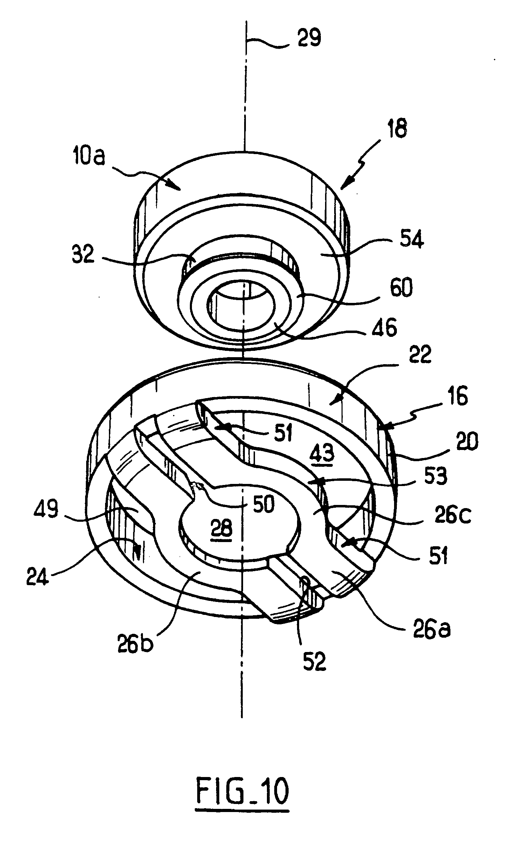

Comme pour les modes de réalisation précités, le verrou 18 et la bride 16 étant initialement séparés comme sur les figures 9 et 10. Pour les assembler, on engage la face inférieure 46 du verrou dans l'orifice 28 de la bride pour mettre en contact la levre d'introduction 60 et le chanfrein 50. On applique un effort de pincement selon l'axe 29, la lèvre d'introduction 60 écarte les deux parties 26b et 26c l'une de l'autre de manière élastique et en glissant sur le chanfrein 50. La déformation élastique cesse lorsque le bras 26a de la bride est introduit dans la gorge 32 du verrou. La bride est alors rendue mobile en rotation par rapport au verrou autour de leur axe commun 29 en étant bloquée à coulissement par rapport au verrou. Dans cette position, la paroi 20 de la bride s'étend sur toute la longueur en regard et à distance du filet 10a du verrou.

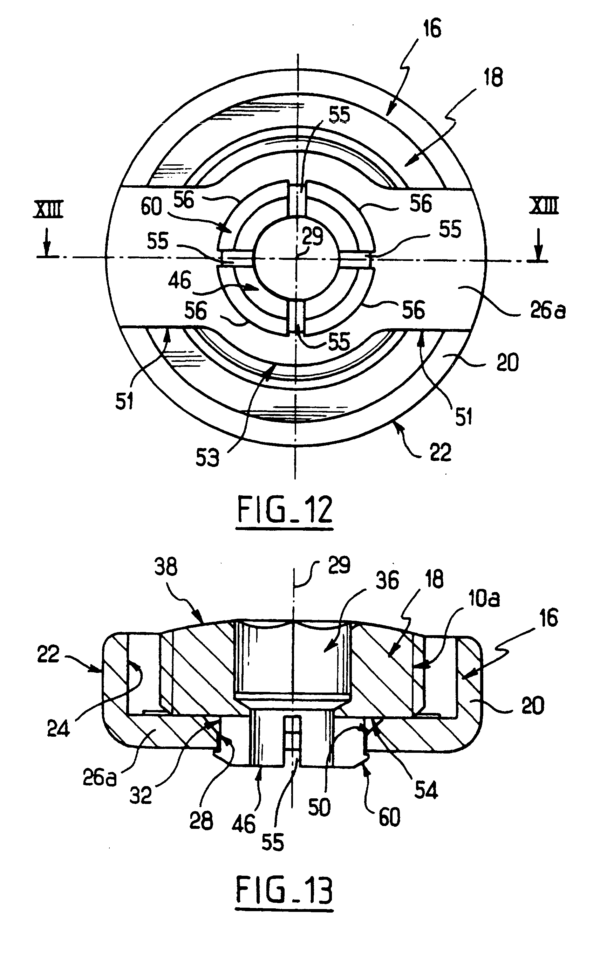

Dans un cinquième mode de réalisation illustré aux figures 12 et 13, le bras 26a est monobloc et ne comprend plus de fente 52. Le verrou 18 est identique au mode de réalisation précédemment décrit sauf en ce qu'il comporte au moins une fente 55 radiale s'étendant de la face d'extrémité 46 du verrou à la paroi 54 du dit verrou et jusqu'au moyeu évidé du verrou. Dans une réalisation préférentielle illustrée, le verrou 18 comporte quatre fentes 55 radiales disposées à 90° les unes des autres dont les axes médians s'intersectent sur l'axe 29. La partie du verrou 18 comportant la gorge 32 et la lèvre d'introduction 60 est ainsi partagée en quatre secteurs 56 identiques. L'assemblage du verrou 18 sur la bride 16 s'effectue de la même façon sauf en ce qui concerne la déformation élastique qui n'est plus supportée par le bras 26a mais par les secteurs 56 du verrou 18. Le montage du dit assemblage sur la vis pédiculaire 2 est identique.

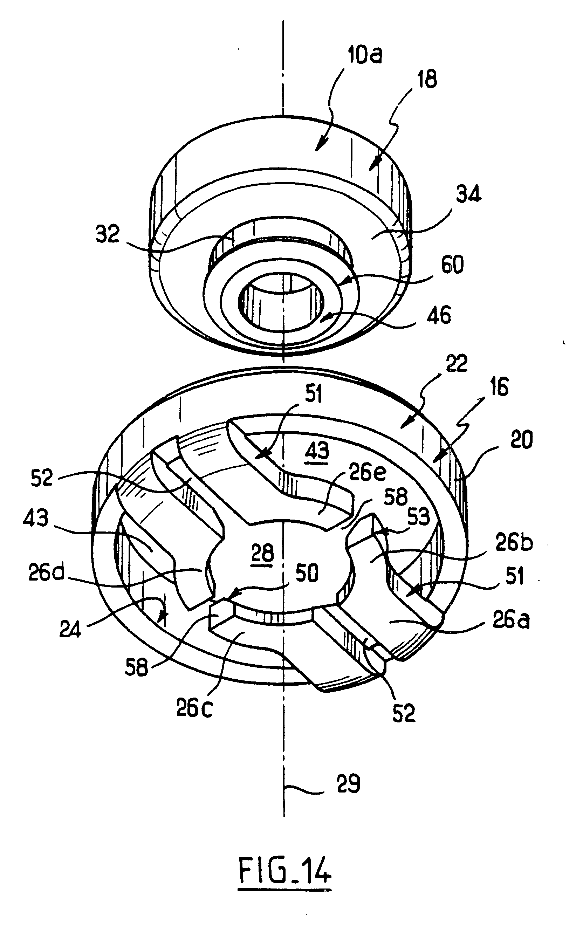

Le sixième mode de réalisation illustré par la figure 14 constitue une variante du quatrième mode de réalisation. Le verrou 18 est inchangé. Sur la bride 16, le bras 26a comporte une fente 58 supplémentaire perpendiculaire à la fente 52 et dont l'intersection des axes médians est située sur l'axe 29. Le bras 26a est constitué de quatre parties 26b, 26c, 26d et 26e symétriques deux à deux en miroir. L'assemblage du verrou sur la bride ainsi que le montage sur la vis pédiculaire 2 sont identiques à ceux décrits pour le quatrième mode de réalisation.

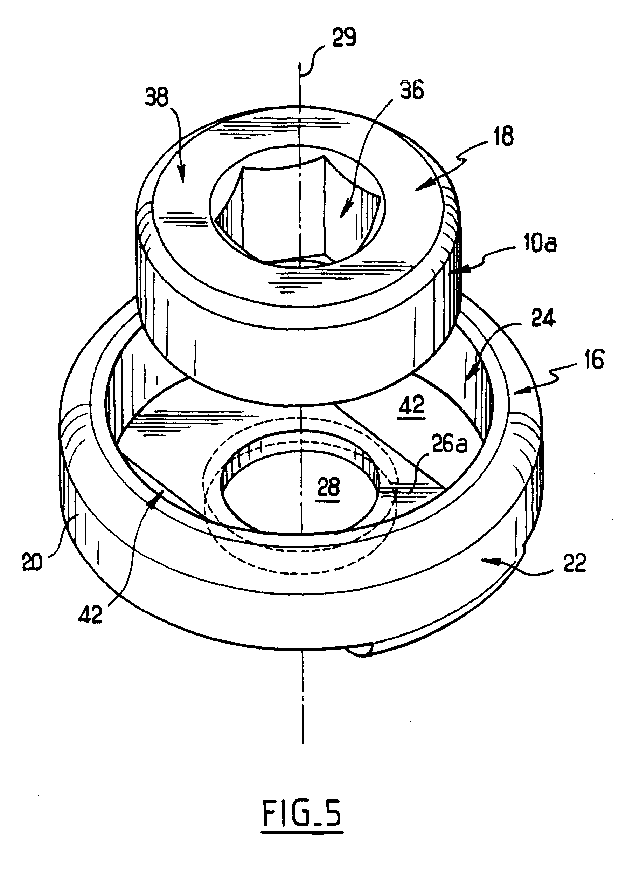

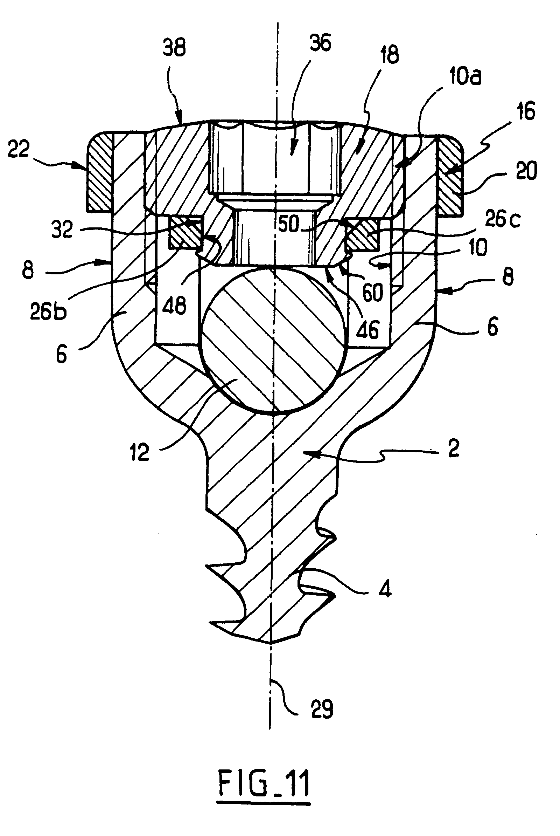

Il est à noter que la figure 11 illustre une variante de montage du système objet de la présente invention sur un organe d'ancrage ne présentant aucune bague ou olive 14 en pièce intermédiaire entre le verrou 18 et la tige 12, comme cela est illustré sur la figure 4.

Bien entendu, on pourra apporter à l'invention de nombreuses modifications sans sortir du cadre de celle-ci.

La gorge pourra être aménagée sur la bride.

Les organes d'ancrage pourront être des crochets.

Le bras pourra s'étendre d'un seul côté du verrou.

Le filet de serrage coopérant avec celui des branches pourra être sur la bride et non sur le verrou. Dans ce cas, la bride assure le vissage sur la tête de vis et le verrou interdit le rapprochement des branches.

## Revendications

1. Système d'ostéosynthèse rachidienne comportant une tige (12), un organe vertébral (2) comprenant deux branches (6) aptes à recevoir la tige entre elles, une bride (16) apte à empêcher l'écartement des branches (6), et un verrou (18) apte à serrer la tige (12) et à être monté mobile à rotation sur la bride (16), l'un (18) parmi la bride (16) et le verrou (18) présentant une gorge (32) apte à recevoir l'autre (16) parmi la bride et le verrou pour le montage du verrou sur la bride, **caractérisé en ce que** le verrou (18) présente un filet de serrage (30 ; 10a) apte à coopérer avec un filet de serrage (10) des branches (6).

2. Système selon la revendication 1, **caractérisé en ce que** la bride (16) et le verrou (18) présentent des filets d'introduction (30, 28) pour introduire dans la gorge (32) l'autre (16) parmi la bride et le verrou.

3. Système selon la revendication 1 **caractérisé en ce que** la bride (16) et/ou le verrou (18) est déformable élastiquement pour introduire dans la gorge (32) le dit autre parmi la bride (16) et le verrou (18).

4. Système selon la revendication 3 **caractérisé en ce que** le verrou (18) comprend une gorge (32).

5. Système selon la revendication 4 **caractérisé en ce que** le verrou (18) comprend au moins une fente (55) au niveau de la gorge (32).

6. Système selon l'une des revendications 1 à 5, **caractérisé en ce que** la bride (16) s'étend en regard du filet de serrage (30 ; 10a) du verrou.

7. Système selon les revendications 2 et 6, **caractérisé en ce que** le filet de serrage du verrou (30) est l'un des filets d'introduction (30, 28).

8. Système selon la revendication 6 ou 7, **caractérisé en ce qu'**il est configuré de sorte que le filet de serrage (30) s'étende entre la gorge (32) et la tige (12).

9. Système selon la revendication 6 ou 7, **caractérisé en ce qu'**il est configuré de sorte que la gorge (32) s'étende entre la tige (12) et le filet de serrage (10a).

10. Système selon l'une quelconque des revendications 1 à 9, **caractérisé en ce que** la bride (16) comprend un bras (26a) apte à s'étendre entre les branches (6) parallèlement à la tige (12) et à participer au montage par gorge (32) du verrou (18) sur la bride (16).

11. Système selon la revendication 10, **caractérisé en ce que** le bras (26a) comprend deux parties aptes à s'étendre de part et d'autre du verrou (18).

12. Système selon la revendication 11, **caractérisé en ce que** les deux parties sont espacées l'une de l'autre.

13. Système selon la revendication 11 ou 12 **caractérisé en ce que** les deux parties sont déformables élastiquement.

14. Système selon la revendication 11, 12 ou 13 **caractérisé en ce qu'**au moins une des deux parties comprend une fente (58).

15. Système selon la revendication 11, **caractérisé en ce que** les deux parties sont reliées l'une à l'autre.

16. Combinaison d'une bride et d'un verrou pour un système d'ostéosynthèse selon la revendication 1, la bride comprenant un collier, le verrou étant apte à être monté mobile à rotation sur la bride, l'un parmi la bride et le verrou présente une gorge apte à recevoir l'autre parmi la bride et le verrou pour le montage du verrou sur la bride, **caractérisée en ce que** le verrou (18) présente un filet de serrage (30 ; 10a) apte à coopérer avec un filet de serrage (10) des branches (6) et la bride et le verrou présentant des moyens de montage pour introduire dans la gorge l'autre parmi la bride et le verrou.

## Claims

1. Spinal osteosynthesis system including a rod (12), a vertebral member (2) comprising two branches (6) which can receive the rod between them, a collar (16) which can prevent the branches (6) from being spaced apart, and a lock (18) which can clamp the rod (12) and can be mounted movably in rotation on the collar (16), either one (18) of the collar (16) or the lock (18) having a groove (32) which can receive the other (16) of the collar or the lock for mounting the lock on the collar, **characterized in that** the lock (18), has a tightening thread (30; 10a) which can cooperate with a tightening thread (10) of the branches (6).

2. System according to Claim 1, **characterized in that** the collar (16) and the lock (18) have introduction threads (30, 28) for introducing the other (16) of the collar or the lock into the groove (32).

3. System according to Claim 1, **characterized in that** the collar (16) and/or the lock (18) is elastically deformable for introducing said other of the collar (16) or the lock (18) into the groove (32).

4. System according to Claim 3, **characterized in that** the lock (18) comprises a groove (32).

5. System according to Claim 4, **characterized in that** the lock (18) comprises at least one slot (55) at the level of the groove (32).

6. System according to one of Claims 1 to 5, **characterized in that** the collar (16) extends opposite the tightening thread (30; 10a) of the lock.

7. System according to Claims 2 and 6, **characterized in that** the tightening thread of the lock (30) is one of the introduction threads (30, 28).

8. System according to Claim 6 or 7, **characterized in that** it is configured in such a way that the tightening thread (30) extends between the groove (32) and the rod (12).

9. System according to Claim 6 or 7, **characterized in that** it is configured in such a way that the groove (32) extends between the rod (12) and the tightening thread (10a).

10. System according to any one of Claims 1 to 9, **characterized in that** the collar (16) comprises an arm (26a) which can extend between the branches (6) parallel to the rod (12) and can participate in the mounting of the lock (18) on the collar (16) via the groove (32).

11. System according to Claim 10, **characterized in that** the arm (26a) comprises two parts which can extend on either side of the lock (18).

12. System according to Claim 11, **characterized in that** the two parts are spaced apart from each other.

13. System according to Claim 11 or 12, **characterized in that** the two parts are elastically deformable.

14. System according to Claim 11, 12 or 13, **characterized in that** at least one of the two parts comprises a slot (58).

15. System according to Claim 11, **characterized in that** the two parts are connected to each other.

16. Combination of a collar and of a lock for an osteosynthesis system according to Claim 1, the collar comprising a flange, the lock being able to be mounted movably in rotation on the collar, either one of the collar or the lock having a groove which can receive the other of the collar or the lock for mounting the lock on the collar, **characterized in that** the lock (18) has a tightening thread (30; 10a) which can cooperate with a tightening thread (10) of the branches (6), and the collar and the lock have means of assembly for introducing the other of the collar or the lock into the groove.

## Patentansprüche

1. Osteosynthesesystem für das Rückgrat, das folgendes umfaßt: eine Stange (12), ein Wirbelorgan (2), das zwei Schenkel (6) umfaßt, die dazu geeignet sind, die Stange zwischen sich aufzunehmen, einen Flansch (16), der dazu geeignet ist, das Auseinanderspreizen der Schenkel (6) zu verhindern, und eine Verriegelung (18), die dazu geeignet ist, die Stange (12) festzuklemmen und drehbar auf dem Flansch (16) angebracht zu werden, wobei einer (18) vom Flansch (16) und der Verriegelung (18) eine Kehle (32) aufweist, die dazu geeignet ist, den anderen (16) vom Flansch und der Verriegelung zum Anbringen der Verriegelung auf dem Flansch aufzunehmen, **dadurch gekennzeichnet, daß** die Verriegelung (18) ein Schraubgewinde (30; 10a) aufweist, das dazu geeignet ist, mit einem Schraubgewinde (10) der Schenkel (6) zusammenzuwirken.

2. System nach Anspruch 1, **dadurch gekennzeichnet, daß** der Flansch (16) und die Verriegelung (18) Einführungsgewinde (30, 28) aufweisen, um den anderen (16) vom Flansch und der Verriegelung in die Kehle (32) einzuführen.

3. System nach Anspruch 1, **dadurch gekennzeichnet, daß** der Flansch (16) und/oder die Verriegelung (18) elastisch deformierbar ist, um den besagten anderen vom Flansch (16) und der Verriegelung (18) in die Kehle (32) einzuführen.

4. System nach Anspruch 3, **dadurch gekennzeichnet, daß** die Verriegelung (18) eine Kehle (32) umfaßt.

5. System nach Anspruch 4, **dadurch gekennzeichnet, daß** die Verriegelung (18) auf der Höhe der Kehle (32) wenigstens einen Schlitz (55) umfaßt.

6. System nach einem der Ansprüche 1 bis 5, **dadurch gekennzeichnet, daß** der Flansch (16) sich gegenüber dem Schraubgewinde (30; 10a) der Verriegelung erstreckt.

7. System nach den Ansprüchen 2 und 6, **dadurch gekennzeichnet, daß** das Schraubgewinde der Verriegelung (30) eines der Einführungsgewinde (30, 28) ist.

8. System nach Anspruch 6 oder 7, **dadurch gekennzeichnet, daß** es so aufgebaut ist, daß das Schraubgewinde (30) sich zwischen der Kehle (32) und der Stange (12) erstreckt.

9. System nach Anspruch 6 oder 7, **dadurch gekennzeichnet, daß** es so aufgebaut ist, daß die Kehle (32) sich zwischen der Stange (12) und dem Schraubgewinde (10a) erstreckt.

10. System nach einem der Ansprüche 1 bis 9, **dadurch gekennzeichnet, daß** der Flansch (16) einen Arm (26a) umfaßt, der dazu geeignet ist, sich zwischen den Schenkeln (6) parallel zur Stange (12) zu erstrecken und bei der Anbringung der Verriegelung (18) auf dem Flansch (16) mittels der Kehle (32) mitwirkt.

11. System nach Anspruch 10, **dadurch gekennzeichnet, daß** der Arm (26a) zwei Teile umfaßt, die dazu geeignet sind sich auf beiden Seiten der Verriegelung (18) zu erstrecken.

12. System nach Anspruch 11, **dadurch gekennzeichnet, daß** die beiden Teile voneinander beabstandet sind.

13. System nach Anspruch 11 oder 12, **dadurch gekennzeichnet, daß** die beiden Teile elastisch verformbar sind.

14. System nach Anspruch 11, 12 oder 13, **dadurch gekennzeichnet, daß** wenigstens einer der beiden Teile einen Schlitz (58) umfaßt.

15. System nach Anspruch 11, **dadurch gekennzeichnet, daß** die beiden Teile miteinander verbunden sind.

16. Kombination eines Flansches und einer Verriegelung für ein Osteosynthesesystem nach Anspruch 1, wobei der Flansch einen Kragen umfaßt, die Verriegelung dazu geeignet ist, drehbar auf dem Flansch angebracht zu werden, und einer vom Flansch und der Verriegelung eine Kehle aufweist, die dazu geeignet ist, zum Anbringen der Verriegelung auf dem Flansch den anderen vom Flansch und der Verriegelung aufzunehmen, **dadurch gekennzeichnet, daß** die Verriegelung (18) ein Schraubgewinde (30; 10a) aufweist, das dazu geeignet ist, mit einem Schraubgewinde (10) der Schenkel (6) zusammenzuwirken, und der Flansch und die Verriegelung Montagemittel aufweisen, um den anderen vom Flansch und der Verriegelung in die Kehle einzuführen.
